# EUROPÄISCHE PATENTSCHRIFT

(11) **EP 1 368 313 B1**
(45) Veröffentlichungstag und Bekanntmachung des Hinweises auf die Patenterteilung: **18.09.2013**
(21) Anmeldenummer: 02742425.8
(22) Anmeldetag: 17.01.2002
(51) Int. Cl.: C07D 211/22

(54) **VERFAHREN ZUR HERSTELLUNG VON NICHT HYDRATISIERTEM FEXOFENADIN-HYDROCHLORID IN FORM EINES NEUEN POLYMORPHS**
METHOD FOR PRODUCING NON-HYDRATED FEXOFENADINE HYDROCHLORIDE AS A NEW POLYMORPHOUS FORM
PROCEDE DE PRODUCTION D'HYDROCHLORURE FEXOFENADINE NON HYDRATE COMME UNE NOUVELLE FORME POLYMORPHE

(30) Priorität: 23.02.2001 CH 329012001
(43) Veröffentlichungstag der Anmeldung: 10.12.2003
(73) Patentinhaber: Cilag AG, 8205 Schaffhausen (CH)
(72) Erfinder: KIRSCH, Volker, CH-8200 Schaffhausen (CH)
(74) Vertreter: Braun, André jr.
(86) Internationale Anmeldenummer: PCT/CH2002/000027
(87) Internationale Veröffentlichungsnummer: WO 2002/066429

(56) Entgegenhaltungen:
- WO-A-00/71124
- WO-A-01/94313
- WO-A-95/31437
- US-A- 4 254 129
- J.GOERDELER: "Ammoniumverbindungen" , HOUBEN-WEYL, METHODEN DER ORGANISCHEN CHEMIE, 4.AUFL., BAND E16A (TEIL2), SEITE 1031 (1990) , GEORG THIEME VERLAG, STUTTGART NEW YORK XP002193831 b) nichtquartäre Ammoniumsalze (Zeilen 7-9)

## Beschreibung

Die vorliegende Erfindung betrifft nicht hydratisiertes Fexofenadin-Hydrochlorid.

Fexofenadin-Hydrochlorid (4-[1-Hydroxy-4-[4-(hydroxydiphenylmethyl)-1-piperidinyl]-butyl]-α,α-dimethyl-phenylessigsäure-Hydrochlorid) weist die folgende Formel (I) 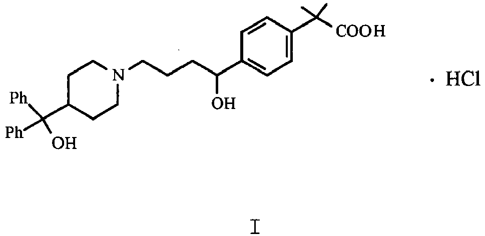 auf und ist unter dem Handelsnamen Allegra^{®} bei der U.S. Food and Drug Administration (FDA) als Antihistaminikum, Antiallergikum und Bronchialdilatator zugelassen.

WO-A-95/31437 beschreibt die Herstellung von hydratisierten Polymorphen, sogenannten Pseudomorphen, des Fexofenadin-Hydrochlorids (Form II und Form IV) und deren Umwandlung in nicht hydratisierte polymorphe Formen (Form I und Form III) durch azeotrope Destillation oder wasserentziehendes Umkristallisieren ("water-minimizing recrystallization").

WO-A-00/71124 beschreibt amorphes, vermutlich nicht hydratisiertes Fexofenadin-Hydrochlorid und dessen Herstellung, wobei als Schlussstufe eine Sprüh- oder Gefriertrocknung vorgenommen wird.

Es wurde nun gefunden, dass man nicht hydratisiertes Fexofenadin-Hydrochlorid in einfacher, direkter Weise, ohne dass Operationen, wie azeotrope Destillation, wasserentziehendes Umkristallisieren, Sprühtrocknung oder Gefriertrocknung notwendig sind, aus Fexofenadin-Base und Chlorwasserstoff herstellen kann, wobei man das nicht hydratisierte Fexofenadin-Hydrochlorid in Form eines neuen Polymorphs ("Form A") erhält, indem man die Fexofenadin-Base in einem niederen Alkylnitril suspendiert, eine Lösung von Chlorwasserstoff in einem niederen Alkanol, in einem Di-nieder-alkylether oder in einem niederen Alkylester einer niederen Alkancarbonsäure zugibt, das Gemisch erhitzt und dann abkühlt, worauf man das nicht hydratisierte Fexofenadin-Hydrochlorid in Form des neuen Polymorphs ("Form A") isoliert.

Die vorstehend als "nieder" bezeichneten Verbindungen und Reste enthalten zweckmässigerweise bis zu acht Kohlenstoffatome. Vorzugsweise verwendet man als niederes Alkylnitril Acetonitril, als niederes Alkanol Methanol, als Di-nieder-Alkylether Diethylether oder Diisopropylether und als niederen Alkylester einer niederen Alkancarbonsäure Ethylacetat.

Die Fexofenadin-Base (II) ist in bekannter Weise aus dem Hydrochlorid des entsprechenden Ketoesters, 4-[1-Oxo-4-[4-(hydroxydiphenylmethyl)-1-piperidinyl]-butyl]-α,α-dimethyl-phenylessigsäure-ethylester, (III) zugänglich.

Das nach dem erfindungsgemässen Verfahren erhältliche Polymorph ("Form A") des Fexofenadin-Hydrochlorids hat einen Schmelzbereich von 153 bis 156 °C (DSC) und wird durch die folgenden XRD-Daten charakterisiert (Tab. 1).

**Tab. 1. XRD-Daten des Fexofenadin Hydrochlorids Form A (d = Netzebenenabstand, die relativen Intensitäten wurden aus dem Pulverdiagramm entnommen, das durch CuK_{α}-Strahlung erhalten wurde).**

| *d* / Å | rel. Intensitäten (I/Iₘₐₓ) / % |
|---|---|
| 11.8 | 55 |
| 11.2 | 30 |
| 7.5 | 50 |
| 6.6 | 30 |
| 5.9 | 20 |
| 5.6 | 70 |
| 5.4 | 20 |
| 4.9 | 65 |
| 4.7 | 100 |
| 4.6 | 35 |
| 4.4 | 40 |
| 4.3 | 100 |
| 4.1 | 40 |
| 4.0 | 30 |
| 3.4 | 40 |

Dieses Polymorph ist neu. Es kann als therapeutischer Wirkstoff verwendet und zu einem Arzneimittel verarbeitet werden, welches den Wirkstoff und einen pharmazeutisch annehmbaren Träger enthält. Dieses Arzneimittel eignet sich als Antihistaminikum, Antiallergikum und/oder Bronchodilatator.

Pharmazeutisch annehmbare Träger, welche bei der Herstellung von Arzneimitteln verwendet werden können, sind allgemein bekannt und jedem Fachmann geläufig.

Durch unterschiedliche Bioverfügbarkeiten, Freisetzungsraten und Löslichkeiten können verschiedene Formen, wie insbesondere neue Polymorphe, eines pharmazeutischen Wirkstoffes von grossem Nutzen für die entsprechenden Patienten sein, da sie eine Verringerung der Dosierung und/oder eine Vergrösserung der Einnahmeintervalle ermöglichen können, wodurch die Kosten der Medikation reduziert werden können.

Die nachfolgenden Beispiele 2-4 sollen die Erfindung illustrieren, ihren Umfang jedoch in keiner Weise einschränken.

### Beispiele

Die XRD-Spektren wurden mit einem computergesteuerten Pulverdiffraktometer-System ADP1700 von Philips mit automatischen Divergenzschlitz und Sekundär-Monochromator (Graphit) aufgenommen. Es wurde die CuK_{α}-Strahlung (λ(CuK_{α1}) = 0.15406 nm und λ(CuK_{α2}) = 0.15444 nm) einer Kupferröhre (40 kV, 30 mA) verwendet und mit Δ(2Θ) = 0.02 bei einer Zählzeit von 3 s im Bereich 1.5°C ≤ 2 Θ ≤ 40° aufgenommen.

Die DSC-Messungen (Differential Scanning Calorimetry) wurden mit einem Gerät METTLER DSC 821^{e} mit einer Starttemperatur von 25 °C, einer Endtemperatur von 250 °C und einer Heizrate von 10 K min⁻¹ aufgenommen. Als Probegefässe dienten Aluminium-Standard-Tiegel mit durchbohrten Deckeln. Die jeweilige Probemenge betrug etwa 5 mg Substanz.

### Beispiel 1

### Synthese der Fexofenadin-Base

30 g Piperidin-Derivat III, 1.7 g Natriumborhydrid und 7.4 g Natriumhydroxid wurden in 200 g Ethanol und 44 g Wasser suspendiert und 3-5 h zum Rückfluss erhitzt und danach mit 10 g Aceton gequenscht. Die Lösungsmittel wurden im Vakuum entfernt und der Rückstand in 200 g Wasser/Aceton (2:1) aufgenommen. Durch Zugabe von Essigsäure wurde ein pH-Wert von 5.8 bis 6.0 eingestellt, wobei die Fexofenadin Base auskristallisierte. Der Niederschlag wurde abfiltriert, mit Wasser gewaschen und im Vakuum bei 60 °C getrocknet, und man erhielt 22 g (83%) des Produktes.

### Beispiel 2

### Synthese des Fexofenadin-Hydrochlorids Form A

86 g Fexofenadin Base wurden in Acetonitril (700 g) suspendiert und bei -10 bis -12 °C mit 30 g einer 20.6 prozentiger Lösung von Chlorwasserstoff in Diisopropylether versetzt. Das Reaktionsgemisch wurde 1 h auf Rückflusstemperatur erhitzt und danach abgekühlt. Das Hydrochlorid wurde durch Filtrieren isoliert, mit Acetonitril gewaschen und bei 100 °C im Vakuum getrocknet. Man erhielt 83 g (90%) des Fexofenadin-Hydrochlorids Form A.

### Beispiel 3

### Synthese des Fexofenadin-Hydrochlorids Form A

10.0 g Fexofenadin-Base wurden in Acetonitril (76 g) suspendiert und bei -10 bis -12 °C mit 1.9 g einer 38.6 prozentiger Lösung von Chlorwasserstoff in Methanol versetzt. Das Reaktionsgemisch wurde 1 h auf Rückflusstemperatur erhitzt und danach abgekühlt. Das Hydrochlorid wurde durch Filtrieren isoliert, mit Acetonitril gewaschen und bei 100 °C im Vakuum. getrocknet. Man erhielt 10.1 g (94%) des Fexofenadin-Hydrochlorids Form A.

### Beispiel 4

### Synthese des Fexofenadin-Hydrochlorids Form A

10.0 g Fexofenadin-Base wurden in Acetonitril (76 g) suspendiert und bei -10 bis -12 °C mit 3.7 g einer 19.5 prozentiger Lösung von Chlorwasserstoff in Ethylacetat versetzt. Das Reaktionsgemisch wurde 1 h auf Rückflusstemperatur erhitzt und danach abgekühlt. Das Hydrochlorid wurde durch Filtrieren isoliert, mit Acetonitril gewaschen und bei 100 °C im Vakuum getrocknet. Man erhielt 9.8 g (91%) des Fexofenadin-Hydrochlorids Form A.

## Patentansprüche

1. Verfahren zur Herstellung von nicht hydratisiertem Fexofenadin-Hydrochlorid in Form eines neuen Polymorphs ("Form A") aus Fexofenadin-Base und Chlorwasserstoff, **dadurch gekennzeichnet, dass** man die Fexofenadin-Base in einem niederen Alkylnitril suspendiert, eine Lösung von Chlorwasserstoff in einem niederen Alkanol, in einem Di-nieder-alkylether oder in einem niederen Alkylester einer niederen Alkancarbonsäure zugibt, das Gemisch erhitzt und dann abkühlt, worauf man das nicht hydratisierte Fexofenadin-Hydrochlorid in Form des neuen Polymorphs ("Form A") isoliert, wobei das nicht hydratisierte Fexofenadin-Hydrochlorid in Form des neuen Polymorphs ("Form A") die folgenden XRD-Daten (gemessen mit einem Pulverdiagrammdiffraktometer mit CuK_{α}-Strahlung) aufweist:
| Netzebenenabstand d / Å | rel. Intensitäten (I/Iₘₐₓ) / % |
|---|---|
| 11.8 | 55 |
| 11.2 | 30 |
| 7.5 | 50 |
| 6.6 | 30 |
| 5.9 | 20 |
| 5.6 | 70 |
| 5.4 | 20 |
| 4.9 | 65 |
| 4.7 | 100 |
| 4.6 | 35 |
| 4.4 | 40 |
| 4.3 | 100 |
| 4.1 | 40 |
| 4.0 | 30 |
| 3.4 | 40 |

2. Verfahren gemäss Anspruch 1, **dadurch gekennzeichnet, dass** man als niederes Alkylnitril Acetonitril verwendet und/oder als niederes Alkanol Methanol verwendet und/oder als Di-nieder-Alkylether Diethylether oder Diisopropylether verwendet und/oder als niederen Alkylester einer niederen Alkancarbonsäure Ethylacetat verwendet.

3. Verfahren zur Herstellung eines Arzneimittels, enthaltend nicht hydratisiertes Fexofenadin-Hydrochlorid in Form eines neuen Polymorphs ("Form A"), **dadurch gekennzeichnet, dass** man die Fexofenadin-Base in einem niederen Alkylnitril suspendiert, eine Lösung von Chlorwasserstoff in einem niederen Alkanol, in einem Di-nieder-alkylether oder in einem niederen Alkylester einer niederen Alkancarbonsäure zugibt, das Gemisch erhitzt und dann abkühlt, worauf man das nicht hydratisierte Fexofenadin-Hydrochlorid in Form des neuen Polymorphs ("Form A") isoliert, wobei das nicht hydratisierte Fexofenadin-Hydrochlorid in Form des neuen Polymorphs ("Form A") die XRD-Daten gemäss Anspruch 1 aufweist.

4. Verfahren nach Anspruch 3, **dadurch gekennzeichnet, dass** das Arzneimittel als Antihistaminikum, Antiallergikum und/oder Bronchodilatator verwendet wird.

## Claims

1. Method for preparing nonhydrated fexofenadine hydrochloride in the form of a novel polymorph ("form A") from fexofenadine base and hydrogen chloride, **characterized in that** the fexofenadine base is suspended in a lower alkyl nitrile, to which a solution of hydrogen chloride in a lower alkanol, in a di(lower alkyl) ether, or in a lower alkyl ester of a lower alkane carboxylic acid is added, the mixture is heated and then cooled, and the nonhydrated fexofenadine hydrochloride in the form of the novel polymorph ("form A") is isolated, wherein the nonhydrated fexofenadine hydrochloride in the form of the novel polymorph ("form A") has the following XRD data (measured with a powder diffractometer using CuK_{α} radiation):
| Lattice spacing d / Å | Relative intensity (I/Iₘₐₓ) / % |
|---|---|
| 11.8 | 55 |
| 11.2 | 30 |
| 7.5 | 50 |
| 6.6 | 30 |
| 5.9 | 20 |
| 5.6 | 70 |
| 5.4 | 20 |
| 4.9 | 65 |
| 4.7 | 100 |
| 4.6 | 35 |
| 4.4 | 40 |
| 4.3 | 100 |
| 4.1 | 40 |
| 4.0 | 30 |
| 3.4 | 40 |

2. Method according to Claim 1, **characterized in that** acetonitrile is used as the lower alkyl nitrile, and/or methanol is used as the lower alkanol, and/or diethyl ether or diisopropyl ether is used as the di(lower alkyl) ether, and/or ethyl acetate is used as the lower alkyl ester of a lower alkane carboxylic acid.

3. Method for preparing a drug containing nonhydrated fexofenadine hydrochloride in the form of a novel polymorph ("form A"), **characterized in that** the fexofenadine base is suspended in a lower alkyl nitrile, to which a solution of hydrogen chloride in a lower alkanol, in a di(lower alkyl) ether, or in a lower alkyl ester of a lower alkane carboxylic acid is added, the mixture is heated and then cooled, and the nonhydrated fexofenadine hydrochloride in the form of the novel polymorph ("form A") is isolated, wherein the nonhydrated fexofenadine hydrochloride in the form of the novel polymorph ("form A") has the XRD data according to Claim 1.

4. Method according to Claim 3, **characterized in that** the drug is used as an antihistamine, antiallergic agent, and/or bronchodilator.

## Revendications

1. Procédé de fabrication de chlorhydrate de fexofénadine non hydraté sous la forme d'un nouveau polymorphe (« Forme A ») de fexofénadine-base et d'acide chlorhydrique, **caractérisé par le fait que** l'on met la fexofénadine-base en suspension dans un alkylonitrile inférieur, on ajoute une solution d'acide chlorhydrique dans un alcanol inférieur, dans un di(alkyl inférieur)éther ou dans un ester d'alkyle inférieur d'un acide alcanecarboxylique inférieur, on chauffe le mélange puis on le refroidit, sur quoi on isole le chlorhydrate de fexofénadine non hydraté sous la forme du nouveau polymorphe (« Forme A »), le chlorhydrate de fexofénadine non hydraté sous la forme du nouveau polymorphe (« Forme A ») présentant les données de XRD suivantes (mesurées au moyen d'un diffractomètre à diagramme de diffraction des rayons X sur poudre à l'aide d'un rayonnement CuK_{α}) ;
| Distance de plan de réseau | Intensités relatives |
|---|---|
| d/A | (I/Iₘₐₓ) / % |
| 11,8 | 55 |
| 11,2 | 30 |
| 7,5 | 50 |
| 6,6 | 30 |
| 5,9 | 20 |
| 5,6 | 70 |
| 5,4 | 20 |
| 4,9 | 65 |
| 4,7 | 100 |
| 4,6 | 35 |
| 4,4 | 40 |
| 4,3 | 100 |
| 4,1 | 40 |
| 4,0 | 30 |
| 3,4 | 40 |

2. Procédé selon la revendication 1, **caractérisé par le fait que**, comme alkylonitrile inférieur, on utilise de l'acétonitrile et/ou, comme alcanol inférieur, on utilise du méthanol et/ou, comme di(alkyl inférieur)éther, on utilise du diéthyléther ou du diisopropyléther et /ou, comme ester d'alkyle inférieur d'un acide alcanecarboxylique inférieur, on utilise de l'acétate d'éthyle.

3. Procédé de fabrication d'un médicament contenant du chlorhydrate de fexofénadine non hydtraté sous la forme d'un nouveau polymorphe (« Forme A »), **caractérisé par le fait que** l'on met la fexofénadine-base en suspension dans un alkylonitrile inférieur, on ajoute une solution d'acide chlorhydrique dans un alcanol inférieur, dans un di(alkyl inférieur)éther ou dans un ester d'alkyle inférieur d'un acide alcanecarboxylique inférieur, on chauffe le mélange puis on le refroidit, sur quoi on isole le chlorhydrate de fexofénadine sous la forme du nouveau polymorphe (« Forme A »), le chlorhydrate de fexofénadine non hydraté sous la forme du nouveau polymorphe (« Forme A ») présentant les données XRD selon la revendication 1.

4. Procédé selon la revendication 3, **caractérisé par le fait que** le médicament est utilisé comme antihistaminique, antiallergique et/ou bronchodilatateur.
